# EUROPÄISCHE PATENTSCHRIFT

(11) **EP 1 814 600 B1**
(45) Veröffentlichungstag und Bekanntmachung des Hinweises auf die Patenterteilung: **22.12.2010**
(21) Anmeldenummer: 05791202.4
(22) Anmeldetag: 20.09.2005
(51) Int. Cl.: A61L 2/00, A61L 2/20, A61L 2/26

(54) **VORRICHTUNG ZUR BEARBEITUNG VON GÜTERN UNTER ZUHILFENAHME EINER ELEKTRISCHEN ENTLADUNG**
DEVICE FOR TREATING GOODS WITH THE AID OF AN ELECTRIC DISCHARGE
DISPOSITIF POUR TRAITER DES MARCHANDISES A L'AIDE D'UNE DECHARGE ELECTRIQUE

(30) Priorität: 12.10.2004 DE 102004049783
(43) Veröffentlichungstag der Anmeldung: 08.08.2007
(73) Patentinhaber: JE PlasmaConsult GmbH, 42119 Wuppertal (DE)
(72) Erfinder: ENGEMANN, Jürgen, 42119 Wuppertal (DE); SCHWABEDISSEN, Axel, 42281 Wuppertal (DE)
(74) Vertreter: Meier, Gerald Heinz
(86) Internationale Anmeldenummer: PCT/DE2005/001651
(87) Internationale Veröffentlichungsnummer: WO 2006/039883

(56) Entgegenhaltungen:
- WO-A-03/059400
- DE-A1- 1 567 738
- US-A- 6 007 770
- US-A1- 2003 108 460
- US-A1- 2004 161 361

## Beschreibung

Die Erfindung betrifft eine Vorrichtung zur Bearbeitung von Gütern unter Zuhilfenahme einer elektrischen Entladung gemäß dem Oberbegriff des Anspruches 1.

Unter der Bearbeitung von Gütern im Sinne der vorliegenden Patentanmeldung wird insbesondere die Konservierung, Desinfektion, Entkeimung oder Sterilisation unterschiedlicher Güter verstanden. Bei den Gütern kann es sich beispielsweise um Lebensmittel, wie Gemüse oder Früchte, aber auch um Kosmetika, medizinische Apparate od. dgl. handeln.

Die Bearbeitung von Gütern im Sinne der vorliegenden Patentanmeldung umfasst aber auch andere Bearbeitungsvorgänge, bei denen z.B. die Güter gebleicht oder oxidiert werden. Schließlich umfasst der Begriff Bearbeitung auch ganz allgemein Oberflächenmodifikationen der Güter.

Insbesondere, aber nicht ausschließlich, betrifft die Erfindung eine Vorrichtung, bei der mit Hilfe einer in der Aufnahmekammer erzeugten elektrischen Entladung Ozon- bzw. UV-Strahlung zum Zweck der teilweisen oder vollständigen Entkeimung von Gütern generiert wird. Diese Art der Bearbeitung kommt insbesondere bei Geräten und Gebrauchsgegenständen aus dem medizinischen und pharmazeutischen Bereich, aber auch bei Kosmetika und Lebensmittel in Betracht.

Medizinische Gebrauchs- und Verbrauchsgüter müssen in der Mehrzahl aller Fälle sterilisiert werden. Hierunter versteht man die Abtötung aller lebenden Mikroorganismen sowie ihrer Ruhestadien (Sporen). Bei pharmazeutischen Verpackungen, Kosmetika und Lebensmitteln, wie Früchten, Obst oder Gewürzen ist häufig eine Keimreduktion (Desinfektion/Konservierung) ausreichend. Zur Sterilisation bzw. Desinfektion stehen eine ganze Reihe von Verfahren zur Verfügung, die ohne Zuhilfenahme einer elektrischen Entladung auskommen und deren Einsatz sich nach dem Material und der Geometrie des Sterilisationsgutes richtet. Das klassische Verfahren ist die Behandlung mit überhitztem Dampf (Autoklavierung, T > 121°C). Aufgrund der zunehmenden Verwendung von hitzeempfindlichen Materialien, z.B. von polymerbasierten Kunststoffen, gibt es einen Bedarf an "kalten" Sterilisations- und Desinfektionsverfahren, die bei niedrigen Temperaturen arbeiten. Dies gilt auch für die Reduktion der Keimbelastung von Lebensmitteln und Kosmetika, die ebenfalls bei max. 50°C behandelt werden müssen.

Bei der Mehrzahl der bekannten Vorrichtungen zur Sterilisation mittels Ozon wird das Ozon nicht in der eigentlichen Behandlungskammer, sondern in einem räumlich davon getrennten Ozongenerator erzeugt und dann über Rohre und Ventile der Behandlungskammer zugeführt. Bekannt sind sowohl Niederdruckverfahren, z. B. gemäß US Pat. 3.719.017 oder gemäß WO 03/039607, die teilweise noch in der klinischen Erprobung sind, aber auch Verfahren, die bei atmosphärischem Druck betrieben werden, z. B. gemäß US Pat. 5.868.999. Die Erzeugung von Ozon erfolgt üblicherweise durch den Betrieb einer mit Hochspannung angeregten "dielektrisch behinderten Entladung" in einem sauerstoffhaltigen Gas. Dieser Typ von Gasentladung wird aufgrund einer vorhandenen elektrischen Isolierung der Elektroden auch Barrierenentladung genannt. Die Barrierenentladung spaltet die Sauerstoffmoleküle in den chemisch sehr aktiven atomaren Sauerstoff, welcher sich sofort mit dem nächsten Sauerstoffmolekül zu Ozon (O₃) verbindet. Diese Reaktion ist sehr schnell und exotherm. Ozon ist nicht stabil und zersetzt sich unter dem Einfluss von Wärme und Katalysatoren (Kontakt mit Gefäßwänden bzw. dem Sterilgut). Die durch den Stromfluss zwischen den Elektroden und die chemische Reaktion entstehende Wärme trägt somit auch direkt zur Zersetzung des Ozons bei, weshalb bei verschiedenen Vorrichtungen zur Sterilisation mittels Ozon ein besonderer Augenmerk darauf gerichtet ist, das erzeugte Ozon bzw. die Entladungselektroden zu kühlen, vgl. z. B. US Pat. 5.002.738 und US 5.169.606.

Allgemein ist zudem festzustellen, dass bei denjenigen Verfahren, die ein gasförmiges wirksames Agens verwenden, dieses Agens in der Regel mehrmals in die gesamte Behandlungskammer eingelassen wird. Die Sterilisation von medizinischen Gütern und Produkten erfolgt in der Regel in einer Verpackung, um eine Rückverkeimung nach Behandlung auszuschließen. Diese Verpackung ist daher semitransparent, d.h. mit Poren versehen, die durchlässig für das wirksame Agens und undurchlässig für bakterielle Sporen und Mikroorganismen ist. Ein geeignetes Material ist z. B. Tyvek^{®} (DuPont Inc.). Somit stellt die Verpackung ein weiteres Hindernis für das wirksame Agens dar und es kann zur vorzeitigen chemischen Zersetzung oder zur Dampfkondensation des wirksamen Agens auf der Außenseite der Verpackung kommen. Bei der Zuleitung des wirksamen Agens vom Generator in die Behandlungskammer kommt es häufig zu einem weiteren Verlust der Konzentration des wirksamen Agens an der Oberfläche der Zuleitungsrohre bzw. der Ventile.

In der WO 03/59400 A1 ist eine Vorrichtung zur Bearbeitung von Gütern, nämlich zur Sterilisierung von Gütern beschrieben, bei der eine elektrische Entladung verwendet wird, um Ozon zur Sterilisierung der Güter zu generieren. Gemäß Fig. 1 dieser Druckschrift ist ein Desinfektionscontainer vorgesehen, an dessen Außenseite zwei flächige Elektroden angeordnet sind. Im Innenraum des Containers ist eine Elektrodenstruktur mit entsprechenden flächigen, zu den äußeren Elektroden parallelen Elektroden angeordnet.

In der US 6.007.770 A ist eine Sterilisationsvorrichtung offenbart, die eine dielektrische Wand, zwei äußere Elektroden und eine innere kapazitiv gekoppelte Elektrode aufweist. Die innere Elektrode liegt lose an der inneren Wand. Bei dieser Ausführungsform ist zwischen der Außenwand und den äußeren Elektroden ein dielektrischer Isolator vorgesehen, der die dielektrische Wand außen umschließt.

Aus der US 2003/018460 A1 ist eine Vorrichtung zur Corona bzw. Ozonerzeugung bekannt, die nur eine einzige Elektrode auf einer Seite einer Wand besitzt. Die Gegenelektrode ist auf der anderen Seite der dielektrischen Wand angeordnet und mit Aussparungen versehen, in denen eine Coronaentladung erzeugt wird. Für die Anordnung in einem geschlossenen Behälter ist diese bekannte Vorrichtung nicht vorgesehen.

Die DE-A 15 67 738 schließlich beschreibt eine Elektrodenanordnung für die Ozonerzeugung, wobei zwei dielektrische Platten und dazwischenliegende elektrisch leitende Bereiche vorgesehen sind, die als Elektroden wirken.

Der Erfindung liegt ausgehend von dem Stand der Technik die Aufgabe zugrunde, eine Vorrichtung zur Bearbeitung von Gütern gemäß dem Oberbegriff des Anspruches 1 derartig weiterzubilden, dass bei einfacher Herstellung eine sicher vorherbestimmbare Betriebsweise möglich wird.

Die Erfindung löst diese Aufgabe mit den Merkmalen des Anspruchs 1 und ist demgemäß dadurch gekennzeichnet, dass bei einer gättungsgemäßen Vorrichtung mit einer Aufnahmekammer, die von einer Wand aus einem dielektrischen Material begrenzt ist, die äußeren Elektroden direkt an der Außenseite der Wand anliegen und die innere Elektrode direkt fest an der Innenseite der Wand aufgeklebt, aufgedruckt, aufgedampft oder im Sputter-Verfahren aufgebracht ist.

Das Prinzip der Erfindung besteht im Wesentlichen darin, an der Außenseite der Wand der Aufnahmekammer zwei Elektroden fest anzuordnen und an der Innenseite der Wand eine Elektrode fest aufzukleben, aufzudrucken, aufzudampfen oder im Sputter-Verfahren aufzubringen.

Die innere Elektrode ist kapazitiv zu den beiden äußeren Elektroden gekoppelt. Dies bedeutet, dass an den beiden äußeren Elektroden eine Wechselspannung anlegbar ist, und an der inneren, kapazitiv gekoppelten Gegenelektrode eine Spannung von den äußeren Elektroden lediglich induziert wird. Die innere Elektrode ist isoliert, also gesondert von den äußeren Elektroden und gesondert von einer Spannungsversorgungseinheit angeordnet. Insbesondere sind keine elektrischen Zuleitungen zu der inneren Elektrode vorgesehen. Durchbrüche in der Wand der Aufnahmekammer für die Durchführung von elektrischen Zuleitungen sind daher entbehrlich.

Dadurch, dass die innere Elektrode fest an der Innenseite der Wand aufgeklebt, aufgedruckt, aufgedampft oder im Sputter-Verfahren aufgebracht ist, ist eine sehr genaue Positionierung der drei Elektroden zueinander möglich.

Die vorgegebene Wandstärke der Wand gibt den Abstand der äußeren Elektroden von der inneren Elektrode sehr genau vor. Damit besteht die Möglichkeit, eine in einem sehr präzisen Maße vorherbestimmbare Oberflächenentladung auszubilden. Diese Oberflächenentfadung kann an einem Randbereich der inneren Elektrode, also unmittelbar innerhalb der Aufnahmekammer, brennen, so dass die elektrische Entladung unmittelbar in der Aufnahmekammer zur Behandlung der Güter brennt. Die infolge der elektrischen Entladung im Falle einer Entkeimung oder Desinfektion zu generierende UV-Strahlung bzw. das Ozon können daher unmittelbar auf die in der Aufnahmekammer angeordneten Güter einwirken.

Als elektrische Entladung erzeugt die erfindungsgemäße Vorrichtung eine sogenannte Oberflächenentladung, die eine spezielle technologische Variante der Barrierenentladung darstellt und als Surface Barrier Discharge bezeichnet wird. Die Oberflächenentladung wurde zuerst von S. Masuda erwähnt (S. Masuda et al., IEEE Trans. Ind. Appl. 24, 223- 231 (1988), US Pat. 4.666.679). Im Gegensatz zur Volumen-Barrierenentladung brennt die Entladung nicht in einem Spalt zwischen den parallel zueinander angeordneten Elektroden, sondern auf der Oberfläche und am Rand zum Dielektrikum einer der Elektroden. Dieser Typ von Barrierenentladung zeichnet sich durch eine besonders hohe Effizienz der Ozonerzeugung aus.

Die erfindungsgemäße Vorrichtung ermöglicht im Falle eines Einsatzes zur Entkeimung und zur Desinfektion eine Bearbeitung der Güter zu relativ geringen Kosten und ohne die Beachtung besonderer Sicherheitsmaßnahmen. Eine Behandlung von beispielsweise Lebensmitteln mit der erfindungsgemäßen Vorrichtung ist zulässig.

Dadurch, dass im Falle einer zur Entkeimung oder Sterilisation eingesetzten Vorrichtung das Ozon und die UV-Strahlung unmittelbar innerhalb der Aufnahmekammer erzeugt wird, ermöglicht die erfindungsgemäße Vorrichtung auch die Behandlung von Produkten mit komplizierterer dreidimensionaler Struktur.

Die erfindungsgemäße Vorrichtung kann eine ausreichend hohe Menge von Ozon und UV-Strahlung über einen ausreichend langen Zeitraum innerhalb einer geschlossenen Verpackung bereitstellen, so dass eine Entkeimung eines temperaturempfindlichen, medizinischen Produktes oder eine Desinfektion oder Teilentkeimung von Kosmetika oder Lebensmitteln, die sich innerhalb der Verpackung befinden, erfolgen kann. Bevorzugt kann die Bearbeitung bei atmosphärischem Druck innerhalb der Verpackung erfolgen, so dass eine kostenaufwändige Vakuumlösung entbehrlich ist. Die Wand der Aufnahmekammer kann dabei auch flexibel ausgebildet sein, so dass eine Vielzahl von Materialien für die Wand der Aufnahmekammer in Betracht kommt und auch auf herkömmliche Verpackungsmaterialien für Güter zurückgegriffen werden kann. Dies ermöglicht auch eine Verwendung der erfindungsgemäßen Vorrichtung als Transportvorrichtung oder als Aufnahmevorrichtung für die Güter.

Die Erzeugung des wirksamen Agens, d.h. z.B. des Ozons in Kombination mit UV-Strahlung geschieht durch eine Oberflächenentladung, z. B. in atmosphärischer Luft innerhalb der Verpackung des zu behandelnden Produktes, also innerhalb der Aufnahmekammer. Hierzu sind außen an der Verpackung, welche z. B. aus einem Kunststoff wie Polyethylen (PE) oder einem anderem Polymer (PA, PVC, PET, ...) hergestellt ist, mindestens zwei metallische Elektroden angebracht, und zwar so, dass kein Luftspalt zwischen Metall und Dielektrikum entsteht. Dies kann im einfachsten Fall durch Aufkleben einer dünnen Metallfolie oder aber durch Aufdrucken (Siebdruck etc.) oder Aufdampfen mittels PVD (= Physical Vapor Deposition) -Verfahren realisiert werden. Die auf der Verpackungsaußenseite angebrachten Elektroden können mit einer Spannungsversorgungseinheit elektrisch verbunden werden, wobei eine Wechselspannung angelegt wird. Die Amplitude der Spannung beträgt vorzugsweise einige kV bis max. 20 kV, die Frequenz beträgt vorzugsweise 1 bis 30 kHz. Der Abstand der Außenelektroden voneinander ist so gewählt, dass es nicht zu einem elektrischen Durchschlag in der Außenluft bei der maximalen angelegten Spannung kommen kann. Alternativ oder ergänzend kann zwischen den beiden äußeren Elektroden noch eine Isolierstrecke oder eine Isolierbarriere zur Verhinderung eines elektrischen Durchschlages angeordnet sein.

Auf der Innenseite der Wand, den äußeren Elektroden gegenüberliegend, ist als Gegenelektrode eine metallische Struktur aufgeklebt, aufgedruckt, aufgedampft oder im Sputter-Verfahren aufgebracht, z. B. ebenfalls aus Kupfer- oder Aluminiumfolie, deren Kanten etwas geringere Abmessungen haben, als diejenigen der äußeren Anregungselektroden. Die Innenelektrode ist eine kapazitiv gekoppelte Gegenelektrode. Diese ist elektrisch "floatend", d.h. es gibt keine metallische Anschlussleitung der Gegenelektrode nach außerhalb der Verpackung. Ebenso wie die äußeren Elektroden ist diese unmittelbar auf die Innenseite der Wand aufgeklebt, aufgedruckt, oder mittels PVD-Verfahren aufgebracht. Sobald die Zündfeldstärke erreicht ist, breitet sich ein Plasma in Form eines Glimmsaums entlang der Kante der Innenelektrode, die zum Dielektrikum gewandt ist, aus. Dieses Plasma erzeugt freie Elektronen, Ionen, Radikale (z. B. atomarer Sauerstoff), sowie UV-Strahlung durch Rekombination von elektronisch angeregten molekularen und atomaren Spezies, insbesondere aber Ozon aus dem Sauerstoff der Luft, in der Aufnahmekammer. Die Menge an Ozon, die erzeugt wird, ist proportional zu der Länge des Glimmsaums. Daher ist es förderlich, wenn die kapazitiv gekoppelte Gegenelektrode eine möglichst lange Randkante aufweist.

Die Plasmaerzeugung wird so lange betrieben, bis der gewünschte Entkeimungsgrad erreicht ist. Dies hängt von der Geometrie und Oberflächenbeschaffenheit des Produktes sowie vom Anwendungsgebiet ab.

Um zu verhindern, dass die z. B. flexibel ausgebildete Verpackung Teile der Oberfläche der zu behandelnden Güter abdeckt und so eine Entkeimung verhindert, kann durch Einblasen von Luft oder einem anderen sauerstoffhaltigen Gasgemisch unmittelbar vor dem Verschließen, z. B. vor dem Verschweißen, der Wand ein leichter Überdruck in der Aufnahmekammer erzeugt werden. Weiterhin kann die Effizienz der Ozonbehandlung dadurch verbessert werden, dass der Grad der Feuchtigkeit innerhalb der Verpackung durch Einsprühen der Verpackung mit einem feinem Wassernebel oder Wassertröpfchen vor dem Verschweißen erhöht wird.

Die erfindungsgemäße Vorrichtung ermöglicht die Erzeugung einer elektrischen Entladung nach Art einer Oberflächenentladung, die hinsichtlich den bekannten Oberflächenentladungen nach Masuda deutlich modifiziert ist. Die neue Elektrodengeometrie ermöglicht eine besonders effiziente Ozonerzeugung, mit einer besonders hohen Ozonkonzentration innerhalb der Aufnahmekammer. Ein Vorteil dieses Verfahrens ist, dass das wirksame Agens (z.B. Ozon und UV-Strahlung) dort erzeugt wird, wo es benötigt wird, also innerhalb der Verpackung. Weiterhin wird keine aufwendige Vakuumtechnologie benötigt, da der Prozess bei Atmosphärendruck stattfinden kann (Kosten und Zeiterspamis).

Das wirksame Agens wird nur innerhalb der Verpackung erzeugt und zersetzt sich automatisch nach dem Abschalten der Spannungsversorgung. Die Halbwertszeit für die Zersetzung von Ozon beträgt ca. 20 Minuten, d.h. lange Ausgasungs- bzw. Belüftungs- und Abpumpzeiten entfallen. Es entstehen keine toxischen Rückstände, da sich O₃ in Sauerstoff bzw. oxidierte Abbauprodukte von organischen Substanzen, d.h. im wesentlichen CO₂ zersetzt.

Eine weiterer Vorteil ist, dass kein Aufwand betrieben werden muss, um ein aktives Agens herzustellen und dem Behandlungsraum zuzuführen. Aufgrund dieser Kostenersparnis ist das Verfahren auch für Produkte mit geringer Wertschöpfung geeignet.

Ein weiterer Vorteil besteht darin, dass ein Netzteil für den notwendigen Frequenzbereich, mit dem die Entladung betrieben werden kann (typischerweise einige kHz) gegenüber einem Hochfrequenzgenerator incl. Abstimmungsnetzwerk zu erheblich günstigeren Kosten hergestellt werden kann.

Ein weiterer Vorteil der Erfindung ist, dass insbesondere flexible Einmalverpackungen verwendet werden können. Gegenüber starren Behältern mit starren Elektroden können bei der erfindungsgemäßen Vorrichtung kostengünstige, recycelbare Verpackungen aus PE-Folie o.ä. Material mit aufgeklebten oder aufgedruckten Elektroden verwendet werden. Die Elektroden können insbesondere Bestandteil von Etiketten sein, die unmittelbar auf die Verpackung aufgeklebt werden. Sowohl die innere Elektrode als auch die äußeren Elektroden können als Bestandteil eines Etiketts ausgebildet sein.

Angemerkt sei, dass die in der vorliegenden Patentanmeldung vornehmlich beschriebene Bearbeitung der Güter eine Entkeimung oder Keimreduktion, Sterilisation, Desinfektion od. dgl. betont. Es kommen allerdings auch eine Reihe weiterer Bearbeitungsarten, und zwar insbesondere Oberflächenmodifikationen ganz unterschiedlicher Art in Betracht, bei denen eine elektrische Entladung eine Rolle spielt. Die Formulierung der vorliegenden Patentanmeldung, wonach die Bearbeitung der Güter unter Zuhilfenahme einer elektrischen Entladung erfolgt, bedeutet dabei insbesondere, dass die elektrische Entladung Sekundärprodukte oder Wirkungen generiert, wie beispielsweise Ozon der UV-Strahlung. Die Formulierung, wonach die Bearbeitung der Güter unter Zuhilfenahme einer elektrischen Entladung erfolgt, schließt aber auch solche Vorrichtungen und Bearbeitungszustände ein, bei der die Bearbeitung der Güter, insbesondere deren Oberfläche, unmittelbar durch die elektrische Entladung, also durch ein Plasma, erfolgt.

Anzumerken ist, dass für den Fall, dass die elektrische Entladung Ozon- oder UV-Strahlung generieren soll, ein gesondertes Agens in die Aufnahmekammer nicht eingebracht werden muss, da sich bei den überaus meisten Verpackungssituationen für die diversen Güter in der Aufnahmekammer Luft befindet. Die erfindungsgemäße Vorrichtung erfordert daher nicht die gesonderte Einbringung eines Agens.

Bei anderen Anwendungsfällen kann die Einbringung eines Agens in die Aufnahmekammer, beispielsweise in Form eines Gasgemisches oder eines Gases durchaus gewünscht und sinnvoll sein.

Die erfindungsgemäße Vorrichtung ist mit einer Spannungsversorgungseinheit verbindbar. Dies bedeutet, dass die Spannungsversorgungseinheit nicht unmittelbarer Bestandteil der Vorrichtung ist, sondern beispielsweise an einem festen Ort angeordnet sein kann. Unterschiedliche Vorrichtungen, die beispielsweise als Transportbehältnisse für Güter ausgebildet sein können, können zur Erzeugung einer elektrischen Entladung und zur Bearbeitung der Güter bedarfsweise mit der Spannungsversorgungseinheit verbunden und nach Durchführung der Bearbeitung von der Spannungsversorgungseinheit auch wieder getrennt also gelöst werden.

Der eigentliche Kniff der Erfindung ist dabei, dass die Wandung der Verpackung die dielektrische Barriere zur Erzeugung einer dielektrisch behinderten Entladung bildet.

Gemäß einer weiteren vorteilhaften Ausgestaltung der Erfindung ist die Wand mit Ausnahme der Elektroden von einer herkömmlichen Verpackung für die Güter gebildet. Die Anordnung der Elektroden zur Konstruktion einer erfindungsgemäßen Vorrichtung erfordert auf diese Weise nur einen sehr geringen zusätzlichen Aufwand.

Gemäß einer weiteren vorteilhaften Ausgestaltung der Erfindung besteht die Wand aus Kunststoff, insbesondere aus PE (Polyethylen), PA (Polyamid), PVC (Polyvinylchlorid), PET (Polyethylenterephthalat) od. dgl. Dies bietet die Möglichkeit, herkömmliche Verpackungsmaterialien zu verwenden.

Gemäß einer weiteren vorteilhaften Ausgestaltung der Erfindung ist die Wand gas-undurchlässig. Dies ermöglicht eine dauerhafte, sichere Unterbringung der Güter innerhalb der Aufnahmekammer, ohne dass, im Falle einer als Entkeimung durchgeführten Bearbeitung, die Gefahr einer erneuten Keimbildung besteht.

Auf besonders vorteilhafte Weise sind die beiden äußeren Elektroden mit Anschlusskontakten einer Spannungsversorgungseinheit verbindbar. Im einfachsten Fall wird eine Spannungsversorgungseinheit mit zwei Anschlussleitungen versehen, wobei die Enden der Anschlussleitungen Kontakte aufweisen, die mit den beiden äußeren Elektroden in eine lösbare elektrische Verbindung bringbar sind. Zugleich, d.h. mit Bewerkstellung einer elektrischen Verbindung, kann auch vorgesehen sein, dass eine mechanische lösbare Verbindung erfolgt.

Für den Fall, dass die äußeren beiden Elektroden auf der Außenseite der Wand fest angeordnet sind, und beispielsweise eine nach außen freiliegende Außenseite aufweisen, können die Anschlusskontakte der Spannungsversorgungseinheit, beispielsweise unter Zuhilfenahme eines Magneten, in unmittelbaren berührenden Kontakt mit den beiden äußeren Elektroden gebracht werden. Dabei ist von besonderer Bedeutung, dass die Wand, die die Aufnahmekammer begrenzt, auch flexibel ausgebildet sein kann.

Bei einer alternativen Ausführungsform der Erfindung sind die äußeren Elektroden mit Steckkontakten versehen, die mit den Anschlusskontakten der Zuleitungen der Spannungsversorgungseinheit unmittelbar lösbar verbindbar sind.

Schließlich ist es auch möglich, die beiden äußeren Elektroden nicht fest an der Außenseite der Wand anzubringen, sondern die beiden äußeren Elektroden, die in diesem Falle mit den Anschlussleitungen der Spannungsversorgungseinheit fest verbunden sind, zur Bearbeitung der Güter unmittelbar an die Außenseite der Wand heranzubewegen. Auch dies kann in einigen Anwendungsfällen sinnvoll sein. Zwar stellt sich auch hier das Problem, dass eine sehr exakte Positionierung der äußeren Elektroden relativ zu der Außenseite der Wand durchgeführt werden muss. Allerdings ist diese Positionierung von außen her durchführbar, wodurch auf einfache Weise gewährleistbar ist, dass zwischen den Elektroden und der Außenseite der Wand kein Luftspalt entsteht. Außerdem können hierzu beispielsweise auf der Außenseite der Wand Markierungen für die äußeren Elektroden zu deren Positionierung vorgesehen sein. Alternativ kann auf der Außenseite der Wand auch eine Markierung vorgesehen sein, die die exakte Position der inneren Elektrode anzeigt.

Vorzugsweise ist jedoch vorgesehen, dass die beiden äußeren Elektroden fest an der Außenseite der Wand angeordnet sind.

Schließlich besteht auch die Möglichkeit, die Aufnahmekammer von einem kasten- oder becherartigen Behältnis auszubilden, welches einen lösbaren Deckel besitzt. Auch kommt die Möglichkeit in Betracht, dass die Vorrichtung eine Unterschale aufweist, wie sie beispielsweise für den Transport von Obst bekannt ist, die auf ihrer Oberseite von einer flexiblen Kunststofffolie verschlossen ist.

Der Zugang kann lediglich einmal verschließbar sein, aber auch wieder öffenbar ausgestaltet sein.

Auf besonders vorteilhafte Weise ist die Wand abschnittsweise oder vollständig flexibel ausgebildet. Für den Fall, dass die Wand vollständig flexibel ausgebildet ist, besteht beispielsweise die Möglichkeit, ein sackartiges Behältnis vorzusehen. Die flexible Ausbildung der Wand, die zumindest teilweise oder abschnittsweise vorgesehen sein kann, birgt den Vorteil eines nur geringen Platzbedarfs zur Aufbewahrung der Vorrichtung, wenn sie nicht benötigt wird. Sie kann dann beispielsweise zusammengelegt oder zusammengefaltet werden. Außerdem kann eine flexible Wand unterschiedliche große Volumina innerhalb der Aufnahmekammer ermöglichen, so dass beispielsweise durch die Erhöhung des Drucks innerhalb der Aufnahmekammer eine Vergrößerung des Volumens der Aufnahmekammer erreichbar ist. Dies ist insbesondere vorteilhaft, wenn Schattenzonen bei der Bearbeitung der Güter, beispielsweise mittels Ozon oder durch UV-Strahlung, vermieden werden sollen, so dass eine Bearbeitung der Güter entlang ihrer gesamten Oberfläche möglich wird.

Alternativ zu einer flexiblen Ausbildung der Wand kann aber auch vorgesehen sein, dass die Wand im Wesentlichen starr ausgebildet ist. Dies ist bei bestimmten Gütern, beispielsweise bei medizinischen Geräten oder Apparaten, unter Umständen vorteilhaft, beispielsweise dann, wenn Aufbewahrungsvorrichtungen des Standes der Technik für diese Güter bereits starre Wände vorsehen. Auch ermöglicht die Erfindung eine Vorrichtung nach Art einer Blisterpackung, die mit zwei äußeren und einer inneren Elektrode versehen ist.

Angemerkt sei, dass die erfindungsgemäße Vorrichtung wenigstens zwei äußere Elektroden und eine innere Elektrode aufweisen muss. Es kann dabei auch vorgesehen sein, noch zusätzliche weitere Elektroden vorzusehen, beispielsweise eine weitere Innenelektrode und ein weiteres Paar zu dieser Innenelektrode angeordneter äußerer Elektroden.

Gemäß einer weiteren vorteilhaften Ausgestaltung der Erfindung ist eine Innenseite oder/und eine Randkante der inneren Elektrode in der Aufnahmekammer frei exponiert. Dadurch, dass zumindest Kanten der inneren Elektrode frei, also offen, angeordnet sind, das heißt, dass diese nicht von einer dielektrischen Materialschicht abgedeckt sind, kann die elektrische Entladung in Form einer Oberflächenentladung generiert werden. Zugleich ermöglicht diese Geometrie, dass die elektrische Entladung unabgeschirmt in der Aufnahmekammer brennt. Die elektrische Entladung wird demgemäß nicht, wie dies beispielsweise in dem Ausführungsbeispiel der Fig. 1 gemäß der WO 03/059400 A1 vorgesehen ist, von einer Abdeckung gegenüber der eigentlichen Aufnahmekammer abgeschirmt. Dies erhöht die Effizienz der Bearbeitung.

Gemäß einer weiteren vorteilhaften Ausgestaltung der Erfindung ist die innere Elektrode relativ zu den beiden äußeren Elektroden derart positioniert und dimensioniert, dass sich bei einer senkrechten Projektion der inneren Elektrode auf die äußeren Elektroden die Kontur der inneren Elektrode im Wesentlichen vollständig innerhalb der Kontur der äußeren Elektroden befindet. Hierzu ist anzumerken, dass vorzugsweise die innere Elektrode und die beiden äußeren Elektroden einander unmittelbar gegenüberliegend angeordnet sind, und lediglich durch die dielektrische Wand der Aufnahmekammer voneinander getrennt sind. Die beiden äußeren Elektroden sind voneinander distanziert angeordnet und werden mit gesonderten Anschlussleitungen mit der Spannungsversorgungseinheit verbunden. Die innere Elektrode weist vorzugsweise zwei Zentren auf, und ist, beispielsweise im Wesentlichen hantelförmig ausgebildet, wobei die beiden Zentren über einen schmalen Steg elektrisch miteinander verbunden sind. Die Fläche der beiden Zentren ist jeweils kleiner als die Fläche der zugehörigen äußeren Elektrode. Würde man die innere Elektrode auf die äußeren Elektroden senkrecht projizieren, so befindet sich die Kontur der inneren Elektrode im Wesentlichen vollständig innerhalb der Kontur der beiden äußeren Elektroden. Die Formulierung "im Wesentlichen" soll dabei berücksichtigen, dass der dünne Verbindungssteg bei dieser Betrachtung unberücksichtigt bleibt.

Gemäß einer weiteren vorteilhaften Ausgestaltung der Erfindung weist die innere Elektrode, insbesondere wenigstens ein Zentrum der inneren Elektrode, eine Kontur mit einer Vielzahl von Richtungsänderungsstellen auf. Diese Ausgestaltung der Erfindung ermöglicht eine besonders lange Randkante der inneren Elektrode, so dass ein besonders langer Glimmsaum der elektrischen Entladung entsteht. Hierdurch wird eine große Menge an Ozon und UV-Strahlung generiert.

Als Richtungsänderungsstelle wird dabei diejenige Stelle bezeichnet, an der man seine Richtung insbesondere abrupt ändern muss, wenn man die Randkante entlangfährt oder an der sich eine Krümmung der Randkante ändert.

Bei der Erfindung ist die innere Elektrode unmittelbar an der Wand angebracht. Auf diese Weise wird einerseits eine besonders einfache Montage der inneren Elektrode an der Wand durch Aufkleben, Aufdampfen, Aufdrucken usw. ermöglicht, ohne dass gesonderte Befestigungselemente erforderlich sind.

Des Weiteren ermöglicht die unmittelbare Anbringung der inneren Elektrode an der Wand allerdings auch eine sehr genaue Positionierung der inneren Elektrode relativ zu den äußeren Elektroden, da der Abstand der inneren Elektrode von den äußeren Elektroden durch die Wanddicke der Wand vorgegeben ist. Die Wanddicke ist dabei aufgrund beispielsweise des Herstellungsprozesses der Wand aber in sehr engen Toleranzen bekannt und vorherbestimmbar. Die physikalischen Parameter für die elektrische Entladung können damit in sehr präzisem Maße vorherbestimmt werden.

Gleichermaßen und unabhängig davon aus denselben Gründen sind die äußeren Elektroden unmittelbar an der Außenseite der Wand angebracht.

Gemäß einer weiteren vorteilhaften Ausgestaltung der Erfindung bestehen die Elektroden aus Metall, insbesondere aus Silber, Gold, Edelstahl, Aluminium oder Kupfer oder aus einer Legierung mit wenigstens einem dieser Metalle. Diese Ausgestaltung der Erfindung berücksichtigt, dass im Falle einer als Ein-Weg-Verpackung ausgebildeten Vorrichtung auch leicht oxidierbare Metalle, wie beispielsweise Kupfer, in Betracht kommen.

Gemäß einer weiteren vorteilhaften Ausgestaltung der Erfindung ist wenigstens eine Elektrode von einer Metallfolie gebildet, die auf die Wand aufgeklebt ist.

Alternativ ist wenigstens eine Elektrode auf die Wand aufgedruckt, aufgedampft oder im Sputter-Verfahren auf die Wand aufgebracht. Dies ermöglicht eine besonders einfache und preiswerte Herstellung der Elektroden.

Die Elektroden können auf der Außenseite der Wand beispielsweise auch als Schriftzug aufgedruckt sein.

Die Elektrode kann auch als Bestandteil eines Etikettes ausgebildet sein, welches an der Wand, insbesondere an einer als Verpackung ausgebildeten Wand, befestigbar, insbesondere aufklebbar, ist.

Das Etikett kann beispielsweise eine Kunststoffumhüllung für die Elektrode aufweisen. Alternativ kann das Etikett auch aus textilem Material oder aus Papier- oder Kartonagematerial gebildet sein, oder eine Kombination unterschiedlicher Materialien, z.B. auch nach Art einer Sandwich-Konstruktion, umfassen.

Die Elektrode kann auch als Bestandteil eines Etiketts von einer Metallfolie gebildet sein. Insbesondere für den Fall, dass das Etikett aus mehreren Materialschichten besteht, kann die Elektrode auf eine der Materialschichten aufgebracht, z.B. aufgeklebt, aufgedampft, aufgedruckt oder aufgesputtert sein.

Das Etikett kann an der Wand durch Kleben oder durch eine andere geeignete Befestigungsart, ggf. auch durch thermisches Verschweißen, befestigt sein. Im Falle einer als Verpackung ausgebildeten Wand, die beispielsweise aus einer Kunststofffolie besteht, ist es besonders vorteilhaft, die die Elektroden aufweisenden Etiketten auf die Verpackung aufzukleben.

Das Etikett kann auf seiner Außenseite eine Information tragen, z.B. eine Identifikations- oder Chargennummer oder einen Barcode, der z.B. aufgedruckt ist. Dem Etikett kann auch eine Anzeigeeinrichtung, z.B. ein Lackmuspapier, zugeordnet sein, die einen Bearbeitungszustand der Güter, z.B. einen erreichten Entkeimungsgrad der Güter, anzeigt. Die Anzeigevorrichtung kann auch als Marker, beispielsweise für Ozon, ausgebildet sein, der bei Erreichung eines bestimmten Bearbeitungszustandes oder nach Durchführung einer chemischen Behandlung einen Farbumschlag zeigt.

Insbesondere für den Fall, dass die Elektrode Bestandteil eines aufklebbaren Elementes, z.B. eines Etikettes, ist, können die Elektroden auf besonders einfache und preiswerte Weise hergestellt und an der Wand befestigt werden. Zugleich kann im Falle einer als Verpackung für die Güter ausgebildeten Wand ein besonders dünnes Verpackungsmaterial gewählt werden. Da die thermische Belastung der Wand bei Betrieb des Plasmas in der Nähe der Elektroden am größten ist, kann durch eine entsprechende Wahl der Dicke des Etikettes die mittlere thermische Belastung der Verpackung in diesem Bereich reduziert werden. Somit kann das Plasma länger betrieben werden, bevor es zu einer Schädigung der Verpackung kommen kann. Durch eine entsprechend dicke Ausbildung der Etiketten kann die Wand selbst sehr dünn, beispielsweise als Verpackungsfolie mit einer Wandstärke von 50 µm, ausgebildet sein, ohne dass die Gefahr einer zerstörerischen thermischen Belastung bei Betrieb des Plasmas besteht.

Besonders vorteilhaft sind sämtliche Elektroden, das heißt die innere als auch die äußeren Elektroden als Bestandteile von Etiketten ausgebildet. Die beiden äußeren Elektroden können dabei von einem gemeinsamen Etikett getragen werden.

Gemäß einer weiteren vorteilhaften Ausgestaltung der Erfindung besteht wenigstens eine Elektrode aus einem elektrisch leitenden und optisch transparenten, also lichtdurchlässigen Material. Hierfür kommt beispielsweise Indium-Zinn-Oxyd (ITO = Indium-Tin-Oxyde) oder ein vergleichbares Material, vorzugsweise ein Metalloxyd in Frage. Ein elektrisch leitendes und optisch transparentes Material bietet die Möglichkeit, auch bei transparenten oder durchsichtigen Behältnissen, wie beispielsweise Glasflaschen oder transparenten Kunststofffolien, äußere und innere Elektroden vorzusehen, ohne dass diese Elektroden in irgendeiner Form optisch auffallen oder gar optisch störend angeordnet sind.

Gemäß einer weiteren vorteilhaften Ausgestaltung der Erfindung ist zwischen den beiden äußeren Elektroden eine Wechselspannung zwischen 0,5 kV und 20 kV mit einer Frequenz zwischen 100 Hz und 10 MHz, vorzugsweise zwischen 1 KHz und 30 KHz, angelegt. Dies ermöglicht eine besonders effiziente Plasmaerzeugung und erfordert lediglich eine preiswerte Spannungsversorgungseinheit.

Die Erfindung soll nachstehend an Hand von Zeichnungen beispielhaft noch näher erläutert werden. Es zeigen:
- Fig. 1: in einer rein schematischen Darstellung das Funktionsprinzip einer Volumenentladung bei einer Vorrichtung des Standes der Technik,
- Fig. 2: in einer gleichermaßen schematischen Querschnittsdarstellung das Funktionsprinzip einer Oberflächenentladung,
- Fig. 3: in einer schematischen Darstellung ein erstes Ausführungsbeispiel der erfindungsgemäßen Vorrichtung,
- Fig. 3a: eine schematische, vergrößerte Ausschnittsdarstellung der Fig. 3,
- Fig. 4: in einer Darstellung ähnlich Fig. 3 ein zweites Ausführungsbeispiel der erfindungsgemäßen Vorrichtung,

- Fig. 5: in einer projizierten Ansicht etwa gemäß Ansichtspfeil V in Fig. 4 die Elektrodenanordnung der Vorrichtung gemäß Fig. 4, und
- Fig. 6: in einer Darstellung gemäß Fig. 5 ein zweites Ausführungsbeispiel einer erfindungsgemäßen Elektrodenanordnung.

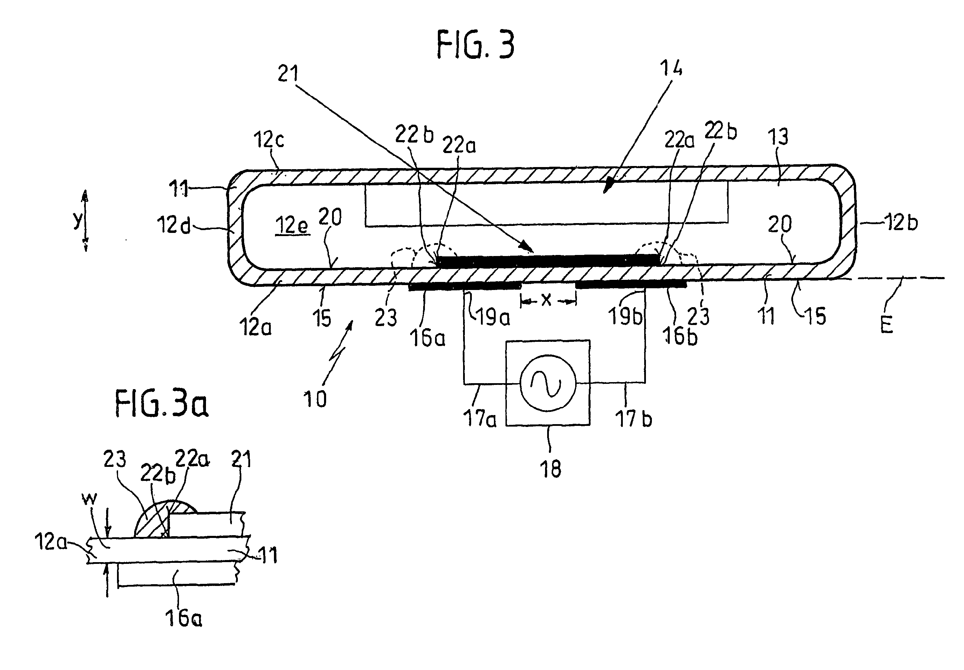

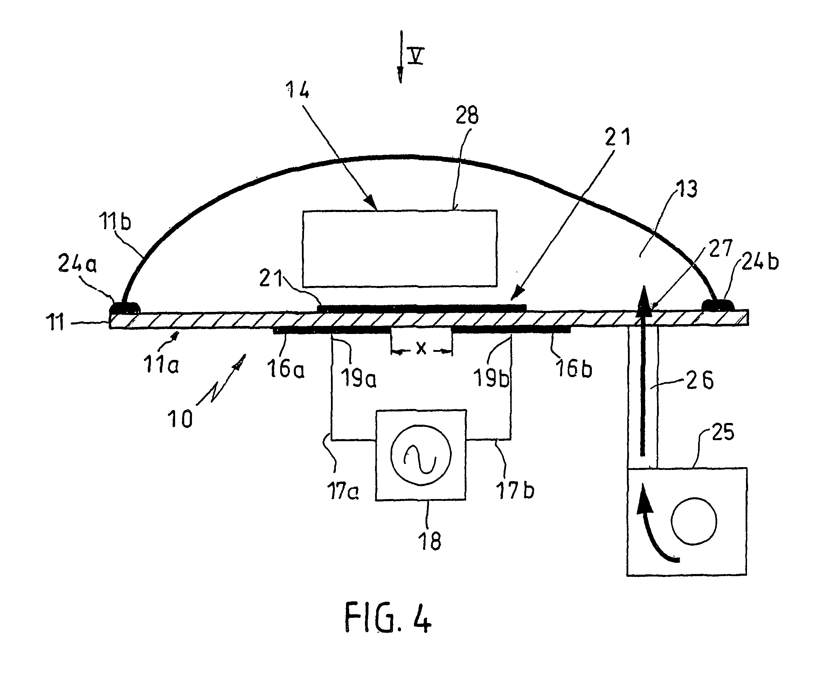

Die erfindungsgemäße Vorrichtung wird anhand der Ausführungsbeispiele der Figuren 3 und 4 in ihrer Gesamtheit mit 10 bezeichnet. Angemerkt sei in diesem Zusammenhang, dass gleiche oder vergleichbare Teile oder Elemente in den unterschiedlichen Figuren der Übersichtlichkeit und der Einfachheit halber mit gleichen Bezugszeichen, teilweise unter Hinzufügung kleiner Buchstaben bezeichnet werden.

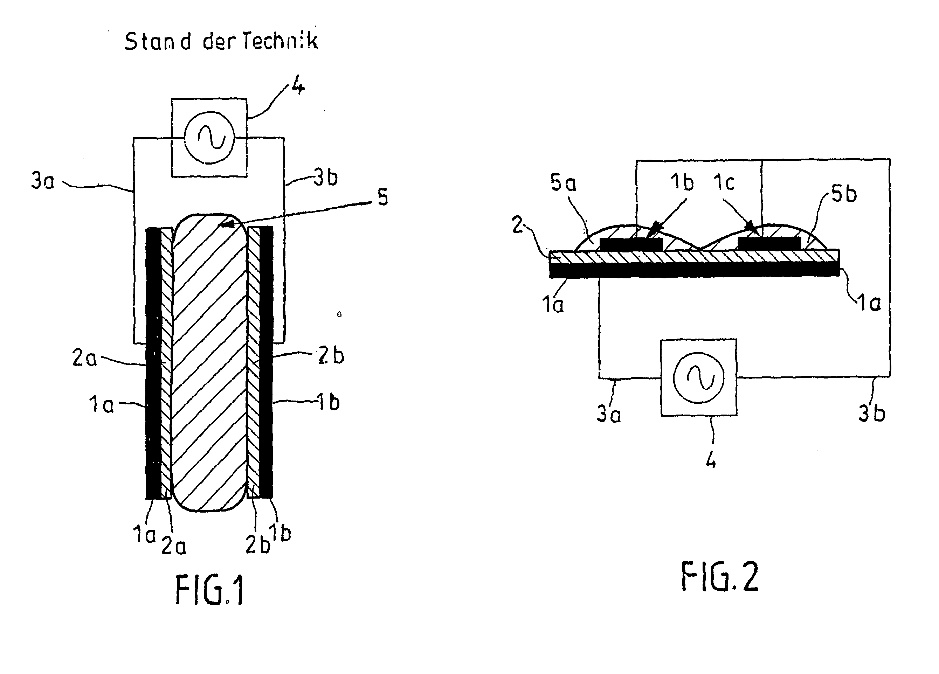

Bevor die erfindungsgemäße Vorrichtung beschrieben wird, soll zunächst anhand der Fig. 1 das Funktionsprinzip einer Volumenentladung beschrieben werden:

Fig. 1 zeigt eine erste Elektrode 1 a, die als Flächenelektrode ausgebildet ist. Dieser Elektrode gegenüberliegend ist eine im Wesentlichen gleichgroße zweite Elektrode 1b angeordnet, so dass eine Anordnung nach Art eines Plattenkondensators entsteht. Jeder Elektrode ist eine dielektrische Barriere 2a, 2b zugeordnet, die jeweils zwischen den beiden Elektroden 1a, 1b angeordnet ist. Die aus einer Schicht oder Platte dielektrischen Materials bestehende dielektrische Barriere 2a ist der ersten Elektrode 1a und die zweite Barriere 2b ist der zweiten Elektrode 1b zugeordnet und jeweils an dieser befestigt. Die beiden Elektroden sind über Anschlussleitungen 3a, 3b mit einer Spannungsversorgungseinheit 4 verbunden. Diese erzeugt eine Wechselspannung, so dass die beiden Elektroden 1a, 1 b auf unterschiedlichem Potential liegen.

Bei geeigneter Wahl von Frequenz und Spannung bildet sich zwischen den dielektrischen Barrieren 2a, 2b eine elektrische Entladung in Form eines Plasmas 5 aus. Dieses Plasma bildet sich, bei vereinfachter Betrachtung, jeweils in den Bereichen zwischen den beiden Elektroden 1 a, 1b, in denen diese den kürzesten Abstand voneinander aufweisen. Das Plasma 5 füllt ein Volumen aus, weshalb man bei dieser Art der elektrischen Entladung auch von einer elektrischen Volumenentladung spricht. Von Bedeutung ist dabei, dass die beiden Elektroden 1 a, 1 b sehr genau zueinander positioniert werden, da jede Abweichung von der Soll-Lage zu unterschiedlichen Abständen und daher auch zu einer unterschiedlichen Ausbildung des Plasmas 5 führt.

Anhand der Fig. 2 soll nun zunächst allgemein das Prinzip der Oberflächenentladung beschrieben werden, das bei der erfindungsgemäßen Vorrichtung zur Anwendung kommt. Wiederum ist eine Spannungsversorgungseinheit 4 vorgesehen, die über Anschlussleitungen 3a, 3b eine Wechselspannung auf eine erste Elektrode 1a und ein zweites Paar von Elektroden 1b, 1c gibt. Die Elektroden 1b, 1c liegen daher auf gleichem Potential, die Elektrode 1a demgegenüber auf einem anderen Potential.

Zwischen der Elektrode 1a und den beiden Elektroden 1b, 1 c ist eine dielektrische Barriere 2 angeordnet Anzumerken ist, dass die beiden Elektroden 1b, 1c voneinander beabstandet sind.

Bei geeigneter Wahl von Frequenz und Spannung bildet sich im Wesentlichen entlang der Kanten der Elektroden 1b, 1c ein Glimmsaum, nämlich eine elektrische Entladung in Form eines Oberflächenplasmas 5a bzw. 5b aus. Da die Entladung im Wesentlichen entlang der Randkanten der Elektroden 1b, 1c stattfindet, spricht man nicht von einem Volumenplasma, sondern von einer Oberflächenentladung.

Anhand der Fig. 3 soll nunmehr die erfindungsgemäße Vorrichtung 10 erläutert werden:

Die Vorrichtung 10 ist gemäß Fig. 3 in einer schematischen Querschnittsansicht dargestellt und umfasst eine Wand 11 mit einem Bodenteil 12a, einem Deckenteil 12c, einem linken Seitenteil 12d und einem rechten Seitenteil 12b. Die Wand 11 umgrenzt und definiert mit ihren Wandteilen 12a, 12b, 12c, 12d eine Aufnahmekammer 13 für beliebige Güter. Fig. 3 zeigt beispielhaft angedeutet ein quaderartiges Gut 14, welches unmittelbar an der Deckenwand 12c anliegt. Das beispielhaft dargestellte Gut 14 kann in diesem Falle beispielsweise an der Deckenwand 12c befestigt sein. Für den Fall, dass das Gut 14 lediglich lose in die Aufnahmekammer 14 eingelegt ist, kann dieses Gut 14 unter Berücksichtigung der Gravitationskraft auf dem Deckenteil 12c der Wand 11 auch aufliegen. In diesem Falle steht Fig. 3 auf dem Kopf, was für die nachfolgende Betrachtung und Erläuterung allerdings unerheblich ist

Ergänzend sei angemerkt, dass die Aufnahmekammer 13 allseitig umschlossen ist. Dementsprechend gehört zu der Wand 11 noch ein nicht dargestelltes Vorderwandteil. Das Hinterwandteil der Wand 11 ist in Fig. 3 angedeutet und wird mit 12e bezeichnet.

Die Wand 11 kann aus einem Kunststoff oder einem beliebigen anderen dielektrischen Material gebildet sein. Vorzugsweise weist die Wand 11 entlang ihren gesamten Umfangs eine konstante Wandstärke W auf. Dies ist allerdings nicht erforderlich.

Die Wand 11 kann aus einem flexiblen Material ausgebildet oder relativ steif ausgebildet sein. Dies ist abhängig vom Einsatzzweck der Vorrichtung 10.

Bei dem Ausführungsbeispiel der Fig. 3 sei zunächst der Einfachheit halber angenommen, dass die Wand 11 aus einem relativ steifen Material besteht.

Fig. 3 lässt erkennen, dass an der Außenseite 15 der Wand 11 eine erste äußere Elektrode 16a und eine zweite äußere Elektrode 16b angebracht und befestigt sind. Anzumerken ist, dass zwischen den beiden Elektroden 16a, 16b und der Außenseite 15 der Wand 11 kein Luftspalt verbleibt, sondern dass die beiden Elektroden 16a, 16b unmittelbar auf der Außenseite 15 der Wand 11 angebracht sind.

Die beiden Elektroden 16a, 16b werden über zugehörige Anschlussleitungen 17a, 17b einer Spannungsversorgungseinheit 18 mit Wechselspannung einer geeigneten Frequenz und einer geeigneten Spannung versorgt. Die freien Enden 19a, 19b der jeweiligen Spannungsversorgungsleitungen 17a, 17b können mit der entsprechenden Elektrode 16a, 16b lösbar in elektrischen Kontakt gebracht werden.

An der Innenseite 20 der Wand 11, die der Außenseite 15 gegenüberliegt, ist eine innere Elektrode 21 angebracht. Die Innenelektrode 21 ist unmittelbar an der Innenseite 20 der Wand 11 befestigt, ohne dass zwischen der Elektrode 21 und der Wand 11 ein Luftspalt verbleibt.

Da die Enden 19a, 19b von den zugehörigen Elektroden 16a, 16b lösbar sind, bildet die Wand 11 mit den daran befestigten Elektroden 16a, 16b, 21 und den innerhalb der Aufnahmekammer 13 angeordneten Gütern (z. B. 14) eine handhabbare Einheit. Die Vorrichtung 10 ist damit als Transportvorrichtung oder Aufbewahrungsvorrichtung für Güter 14 geeignet. Sie kann bei Bedarf, dann wenn es erforderlich ist und eine Bearbeitung der innerhalb der Aufnahmekammer 13 befindlichen Güter 14 erfolgen soll, mit den Anschlussleitungen 17a, 17b einer Spannungsversorgungseinheit 18 verbunden werden.

Befindet sich die Vorrichtung in Betrieb, bildet sich entlang der Kanten 22a, 22b der inneren Elektrode 21 eine elektrische Entladung aus, und zwar eine Oberflächenentladung, also ein Plasma nach Art eines Glimmsaumes. Der entstehende Glimmsaum ist gepunktet in Fig. 3 angedeutet und dort mit 23 bezeichnet. Der Übersichtlichkeit halber ist in Fig. 3a, die in einer schematischen Darstellung eine vergrößerte Ausschnittsdarstellung des bezüglich Fig. 3 linken Randbereiches der inneren Elektrode 21 und der äußeren Elektrode 16a zeigt, der Glimmsaum 23 schraffiert schematisch dargestellt. Das erzeugte Plasma nach Art eines Glimmsaumes entlang der Randkanten ist ein filamentäres Plasma, also kein APG-Plasma, mithin kein Glow-Plasma. Durch Erzeugung einer elektrischen Entladung 23 innerhalb der Aufnahmekammer 13 wird Ozon und UV-Strahlung generiert. Ozon und UV-Strahlung werden im Folgenden als Agens bezeichnet. Dieses Agens kann mit den in der Aufnahmekammer 13 befindlichen Gütern 14 zusammenwirken und diese Güter beispielsweise entkeimen, desinfizieren, sterilisieren od. dgl. Auch andere Bearbeitungsarten, je nach dem um welche Güter es sich handelt, und in Abhängigkeit von der innerhalb der Aufnahmekammer 13 befindlichen Gas oder Gasmischungen kommen in Betracht. So ist auch ein Bleichen, ein Oxidieren oder eine sonstige Oberflächenmodifikation der in der Aufnahmekammer 13 befindlichen Güter 14 denkbar.

Anzumerken ist, dass die innere Elektrode 21 eine kapazitiv gekoppelte Gegenelektrode zu den beiden äußeren Elektroden 16a, 16b darstellt. Die innere Elektrode 21 ist somit nicht mit irgendwelchen außerhalb der Aufnahmekammer 13 angeordneten Spannungsversorgungsleitungen einer Spannungsversorgungseinheit verbunden. Die innere Elektrode 21 ist davon völlig unabhängig. In ihr wird eine Spannung ausschließlich über die äußeren Elektroden 16a, 16b induziert.

Dadurch, dass die innere Elektrode 21 und die beiden äußeren Elektroden 16a, 16b unmittelbar an der Wand 11 befestigt sind, ist die Position der Elektroden 16a, 16b, 21 relativ zueinander eindeutig vorherbestimmt. Insbesondere der in Richtung des Doppelpfeiles y gemäß Fig. 3 festzulegende Abstand der Elektroden voneinander wird aufgrund der Wandstärke w (Fig. 3a) der Wand 11 in diesem Bereich präzise definiert. Damit kann die Obertlächenentladung 23 auch in einem sehr exakten Maße vorherbestimmt werden.

Anzumerken ist, dass die Möglichkeit besteht, die Wandstärke w der Wand 11 der Vorrichtung 10 an die Erfordernisse der gewünschten Oberflächenentladung anzupassen. Andererseits kann durch eine Änderung der Elektrodengeometrie eine vorhandene und nicht änderbare Wandstärke w der Kammerwand 11 berücksichtigt werden. Die beiden äußeren Elektroden 16a, 16b sind entlang der Ebene E um den Abstand x voneinander beabstandet. Der Abstand x ist so gewählt, dass ein Durchschlag zwischen Elektroden 16a, 16b verhindert wird. Erforderlichenfalls kann auch zwischen den beiden Elektroden 16a und 16b ein in Fig. 3 nicht dargestellter Isolator angeordnet sein.

Fig. 4 zeigt ein zweites Ausführungsbeispiel der erfindungsgemäßen Vorrichtung 10, bei der die Wand 11 der Vorrichtung aus einem ersten Wandabschnitt 11a und einem zweiten Wandabschnitt 11b besteht. Der erste Wandabschnitt 11a kann beispielsweise relativ steif oder starr ausgebildet sein und eine Art Trägerplatte bereitstellen. Der zweite Wandabschnitt 11 b kann von einer flexibleren Verpackung, beispielsweise von einer Folie bereitgestellt sein. Die beiden Wandungsteile 11a, 11b können im Bereich von Verbindungsbereichen 24a, 24b miteinander fest verbunden, beispielsweise verschweißt sein, so dass eine vollständig geschlossene Aufnahmekammer 13 für Güter 14 bereitgestellt wird. Wiederum ist in Fig. 4 ein Gut 14 in Form eines Rechtecks lediglich sehr schematisch angedeutet.

Die Anordnung der Elektroden 16a, 16b und 21 ist vergleichbar zu der Elektrodenanordnung gemäß Fig. 3, so dass auf deren Beschreibung an dieser Stelle verzichtet sein soll. Als Besonderheit ist bei dem Ausführungsbeispiel gemäß Fig. 4 der Vorrichtung 10 eine Gebläseeinheit 25 zugeordnet, die über einen Anschlussstutzen 26 und eine Durchlassöffnung 27 in der Trägerplatte 11a hindurch Luft in die Aufnahmekammer 13 hineinbläst. Die Durchlassöffnung 27 ist nach Art eines Ventils wiederverschließbar, so dass nach Bearbeitung der Güter 14 eine vollständige Gasdichtigkeit bzw. Gasundurchlässigkeit der Vorrichtung 10 erreichbar ist.

Das Einblasen von Luft ermöglicht, dass sich die flexible Wand 11b von der Oberseite 28 des Gutes 14 abheben kann, so dass das Gut 14 allseitig von Luft umgeben ist. Dies fördert eine homogene Bearbeitung der Oberflächen der Güter 14.

Angemerkt sei, dass die Fig. 4 lediglich schematisch zu verstehen ist, da aufgrund der Schwerkraft das Gut 14 nahezu zwangsläufig mit Werkstoffbereichen an den Innenseiten des Wandabschnittes 11a aufliegt. Ergänzend sei angeführt, dass die Vorrichtung während der Bearbeitung bewegt werden kann, beispielsweise durch ein Rütteln oder Schütteln, so dass dafür gesorgt wird, dass jeweils unterschiedliche Bereiche der Güter 14 an den Innenseiten der Wand 11 aufliegen, so dass ebenfalls eine homogene oder homogenere Bearbeitung der Güter möglich wird.

Alternativ zu dem Einblasen von Gas in die Kammer gemäß dem Ausführungsbeispiel der Fig. 4 und durch Erzeugung eines Überdrucks in der Aufnahmekammer 13 kann der gleiche Effekt dadurch erreicht werden, dass außerhalb der Wand 11 ein Unterdruck erzeugt wird.

Angemerkt sei schließlich auch, dass über das Gebläse 25, die Anschlussstrecke 26 und die Durchlassöffnung 27 auch besondere Gase oder Gasmischungen, teilweise auch weitere Agens, in die Aufnahmekammer 13 eingeführt werden können. Dies ist insbesondere bei Oberflächenmodifikationen von Bedeutung. Auch bei der Vorrichtung 10 gemäß Fig. 4 ist ein Lösen der Enden 19a, 19b der Anschlussleitungen 17a, 17b von den äußeren Elektroden 16a, 16b vorgesehen, so dass die Vorrichtung 10 lösbar mit der Spannungsversorgungseinheit 18 verbindbar ist und eine handhabbare Einheit darstellt, die als Transportvorrichtung oder Aufnahmevorrichtung für die Güter 14 dienen kann.

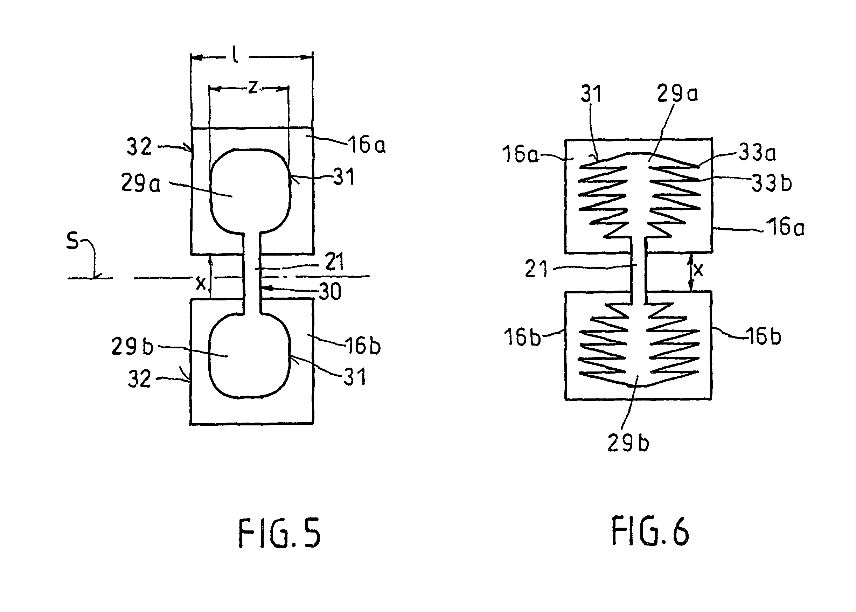

Anhand der Fig. 5 und 6 sei nunmehr noch die Geometrie der Elektroden erläutert:

Die Figuren 5 und 6 zeigen jeweils eine Projektionsansicht etwa entlang dem Ansichtspfeil 5 in Fig. 4, wobei lediglich die innere Elektrode 21 und die beiden äußeren Elektroden 16a, 16b dargestellt sind. Mit anderen Worten sind die Wandung 11b, das Gut 14 und der Wandungsabschnitt 11a der Übersichtlichkeit halber weggelassen.

Fig. 5 zeigt, dass die innere Elektrode 21 im Wesentlichen hantelartig ausgebildet ist und ein erstes Zentrum 29a und ein zweites Zentrum 29b aufweist, die über einen schmalen Verbindungssteg 30 miteinander verbunden sind. Die beiden äußeren Elektroden 16a, 16b sind im Wesentlichen quadratisch ausgebildet und voneinander um den Abstand x voneinander beabstandet. Sowohl die innere Elektrode 21 als auch die beiden äußeren Elektroden 16a, 16b sind symmetrisch angeordnet entlang einer Symmetrieebene S.

Aus Fig. 5 wird deutlich, dass jede der äußeren Elektroden 16a, 16b ein Quadrat mit der Kantenlänge I darstellt. Jedes Zentrum 29a, 29b der inneren Elektrode 21 entspricht im Wesentlichen einem Quadrat mit abgerundeten Ecken, wo das Quadrat eine Kantenlänge Z aufweist. Z ist kleiner als I, so dass die projizierte Fläche eines jeden Zentrums 29a, 29b innerhalb der Konturen 32 einer äußeren Elektrode 16a, 16b liegt. Die Kontur der inneren Elektrode 21 ist mit 31 bezeichnet, die Kontur der äußeren Elektrode 16a, 16b ist mit 32 bezeichnet.

Da die Kontur 31 der inneren Elektrode 21, bis auf die Kontur des Verbindungssteges 30, vollständig innerhalb der Kontur 32 der äußeren Elektroden 16a, 16b liegt, entsteht ein Glimmsaum 23 (Fig. 3) innerhalb der Aufnahmekammer 13. Von Bedeutung ist, dass die Länge des Glimmsaums im Wesentlichen proportional zu der Länge der entsprechenden Randkante 31, 22a, 22b der inneren Elektrode 21 ist. Fig. 5 verdeutlicht anhand der Hantelstruktur der inneren Elektrode 21 eine Randkante 31, die allerdings nicht besonders lang ausgeprägt ist. Ein weiteres Ausführungsbeispiel der inneren Elektrode 21 gemäß Fig. 6 zeigt eine innere Elektrode 21 nach Art einer Doppel-Tannenbaum-artigen oder Farnblatt-artigen Struktur, bei der die Randkante oder Kontur 31 der inneren Elektrode 21 mit einer Vielzahl von Richtungsänderungsstellen 33a, 33b versehen ist. Dies sind Stellen, an denen man beim Entlangfahren entlang der Randkante 31 seine Richtung ändert, also beispielsweise die Krümmung von einer Rechtskurve in eine Linkskurve wechselt, oder Stellen, an denen sich die Richtung abrupt ändert, nach Art einer Unstetigkeitsstelle, insbesondere zick-zack-artig. Mithin entstehen bei der inneren Elektrode 21 gemäß Fig. 6 besonders lange Randkanten 31, die die Erzeugung eines besonders langen Glimmsaumes 23 und damit eine Erzeugung eines großen Anteils an Ozon oder UV-Strahlung gewährleisten.

Von besonderer Bedeutung ist, dass die Elektroden auch gezackt oder alternativ auch serpentinenförmig ausgebildet sein können. Die Strukturierung der Elektrodenkontur ist dabei nur entlang einer Ebene E von Bedeutung.

Nicht dargestellte innere Elektroden 21 können darüber hinaus auch eine Reihe von Öffnungen aufweisen. So kann beispielsweise jedes Zentrum 29a, 29b einer Elektrode 21 gemäß Fig. 5 mit einer Vielzahl von löcherartigen Öffnungen versehen sein, so dass die Kantenlänge der Randbereiche deutlich erhöht wird. Hierdurch kann die Ozon-Erzeugung weiter verbessert werden.

Die Elektrodendimensionen können im Millimeter- oder im Zentimeter-Bereich liegen. Je kleiner die Dimensionen der Vorrichtung 10 insgesamt sind, umso kleiner können auch die Elektrodenflächen gewählt werden.

Die Ausführungsbeispiele der Figuren 3 und 4 betreffen sowohl Vorrichtungen 10, die eine flexible Wand aufweisen, als auch Vorrichtungen, die eine im Wesentlichen steif ausgebildete Wand aufweisen. Bei einer flexiblen Vorrichtung ist es beispielsweise also denkbar, die Wand von einem flexiblen Kunststoffsack oder -beutel zu bilden, der über einen Reißverschluss eine Zugangsöffnung zu der Aufnahmekammer 13 bereitstellt. Auf diese Weise können beispielsweise in einer Arztpraxis medizinische Geräte ohne Weiteres in die Aufnahmekammer 13 eingebracht werden und die Aufnahmekammer über den Reißverschluss verschlossen werden. Anschließend kann die Bearbeitung der medizinischen Geräte, beispielsweise in Form einer Desinfektion, erfolgen.

Alternativ kann die Wand 11 auch aus relativ steifem Material gebildet sein, wobei eine Zugangsöffnung für die Aufnahmekammer von einer Tür, einer Klappe, einem Deckel od. dgl. gebildet ist.

Schließlich kommen auch solche Vorrichtungen in Betracht, bei denen die Kammer dauerhaft fest und vollständig von der Wand 11 verschlossen ist, wie es beispielsweise in Fig. 4 angedeutet ist, wo Verschweißstellen 24a, 24b angedeutet sind. In diesem Falle kann beispielsweise eine herkömmliche Transportverpackung für Lebensmittel von einer Folienschweißmaschine od. dgl. verschlossen werden. Die erfindungsgemäße Vorrichtung ermöglicht dann eine Bearbeitung der bereits vollständig verpackten Güter, und somit eine Bearbeitung zu einem Zeitpunkt, an dem die Kammerwand die Aufnahmekammer vollständig verschließt.

Der Feuchtigkeitsgehalt innerhalb der Aufnahmekammer 13 kann vor dem Verschließen der Wand 11 und damit vor dem Bearbeiten der Güter beispielsweise auf einfache Weise dadurch erhöht werden, dass die Innenseite der Wand 11 mit Wasserdampf besprüht wird. Ein erhöhter Wasserdampfanteil kann die Ozonerzeugung effizienter gestalten.

Bei allen Ausführungsbeispielen ist von besonderer Bedeutung die Wahl der Abstände der Elektroden 16a, 16b und 21 voneinander und gleichermaßen auch die Wahl der Spannung, so dass es zu einer Gasentladung ausschließlich innerhalb der Aufnahmekammer 13 und nicht außerhalb der Aufnahmekammer 13 kommt.

## Patentansprüche

1. Vorrichtung (10) zur Bearbeitung von Gütern (14) unter Zuhilfenahme einer elektrischen Entladung in einer Aufnahmekammer (13) für die Güter (14), die von einer Wand (11, 11a, 11b, 12a, 12b, 12c, 12d, 12e) aus einem dielektrischen Material begrenzt ist, an deren Außenseite (15) wenigstens zwei äußere Elektroden (16a, 16b) angeordnet sind
und wobei an der Innenseite (20) der Wand (11, 11a, 11b, 12a, 12b, 12c, 12d, 12e) wenigstens eine kapazitiv zu den beiden äußeren Elektroden (16a, 16b) gekoppelte Gegenelektrode (21) angeordnet ist,
**dadurch gekennzeichnet,**
**dass** die beiden äußeren Elektroden (16a, 16b) direkt an der Außenseite (15) anliegen
und **dass** die innere Elektrode (21) direkt fest an der Innenseite (20) aufgeklebt, aufgedruckt, aufgedampft oder im Sputter-Verfahren (PVD) aufgebracht ist.

2. Vorrichtung nach Anspruch 1,
**dadurch gekennzeichnet,**
**dass** wenigstens eine Elektrode (16a, 16b, 21) als Bestandteil eines Etikettes ausgebildet ist.

3. Vorrichtung nach Anspruch 1,
**dadurch gekennzeichnet,**
**dass** wenigstens eine Elektrode (16a, 16b, 21) von einer Metallfolie gebildet ist, die auf die Wand aufgeklebt ist.

4. Vorrichtung nach Anspruch 1,
**dadurch gekennzeichnet,**
**dass** wenigstens eine der äußeren Elektroden (16a, 16b) auf die Wand (11) aufgedruckt, aufgedampft oder im Sputter-Verfahren (PVD) aufgebracht ist.

5. Vorrichtung nach einem der Ansprüche 1 bis 4,
**dadurch gekennzeichnet,**
**dass** wenigstens eine Elektrode (16a, 16b, 21) aus einem optisch transparenten und elektrisch leitendem Material besteht, beispielsweise aus Indium-Zinn-Oxyd (ITO).

6. Vorrichtung nach einem der Ansprüche 1 bis 5,
**dadurch gekennzeichnet,**
**dass** die innere Elektrode (21) im Wesentlichen hantelförmig ausgebildet ist.

7. Vorrichtung nach einem der Ansprüche 1 bis 6,
**dadurch gekennzeichnet,**
**dass** die innere Elektrode (21) zwei Zentren (29a, 29b) aufweist, die miteinander verbunden sind.

8. Vorrichtung nach Anspruch 7,
**dadurch gekennzeichnet,**
**dass** die Fläche eines Zentrums (29a, 29b) kleiner ist als die Fläche einer zugehörigen, gegenüberliegenden äußeren Elektrode (16a, 16b).

9. Vorrichtung nach einem der Ansprüche 1 bis 8,
**dadurch gekennzeichnet,**
**dass** die innere Elektrode (21) eine Kontur mit einer Vielzahl von Richtungsänderungsstellen (33a, 33b) aufweist.

## Claims

1. Device (10) for treating goods (14) with the assistance of an electrical discharge in a receiving chamber (13) for the goods (14), the chamber being bounded by a wall (11, 11 a, 11 b, 12a, 12b, 12c, 12d, 12e) of a dielectric material, at the outer side (15) of which at least two outer electrodes (16a, 16b) are arranged, and wherein at least one counterelectrode (21) capacitively coupled with the two outer electrodes (16a, 16b) is arranged at the inner side (20) of the wall (11, 11a, 11b, 12a, 12b, 12c, 12d, 12e), **characterised in that** the two outer electrodes (16a, 16b) bear directly against the outer side (15) and that the inner electrode (21) is directly firmly glued to, printed on, vapour-deposited on or applied in a sputtering process (PVD) to the inner side (20).

2. Device according to claim 1, **characterised in that** at least one electrode (16a, 16b, 21) is constructed as a component of a label.

3. Device according to claim 1, **characterised in that** at least one electrode (16a, 16b, 21) is formed by a metal foil glued to the wall.

4. Device according to claim 1, **characterised in that** at least one of the outer electrodes (16a, 16b) is printed on, vapour-deposited on or applied in a sputtering process (PVD) to the wall (11).

5. Device according to any one of claims 1 to 4, **characterised in that** at least one electrode consists of an optically transparent and electrically conductive material, for example of indium-tin-oxide (ITO).

6. Device according to any one of claims 1 to 5, **characterised in that** the inner electrode (21) is of substantially dumbbell-shaped construction.

7. Device according to any one of claims 1 to 6, **characterised in that** the inner electrode (21) has two centres (29a, 29b) which are connected together.

8. Device according to claim 7, **characterised in that** the area of a centre (29a, 29b) is smaller than the area of an associated opposite outer electrode (16a, 16b).

9. Device according to any one of claims 1 to 8, **characterised in that** the inner electrode (21) has a profile with a plurality of directional change points (33a, 33b).

## Revendications

1. Dispositif (10) pour traiter des produits (14) avec une décharge électrique dans une chambre de réception (13) pour les produits (14), cette chambre étant délimitée par une paroi (11, 11a, 11b, 12a, 12b, 12c, 12d, 12e) en une matière diélectrique et dont le côté extérieur (15) comporte au moins deux électrodes extérieures (16a, 16b), et
le côté intérieur (20) de la paroi (11, 11a, 11b, 12a, 12b, 12c, 12d, 12e) comporte au moins une contre-électrode (21) couplée de manière capacitive avec les deux électrodes extérieures (16a, 16b),
dispositif **caractérisé en ce que**
les deux électrodes extérieures (16a, 16b) sont appliquées directement contre le côté extérieur (15), et
l'électrode intérieure (21) est collée, imprimée, déposée à la vapeur ou par un procédé de pulvérisation (PVD) directement sur le côté intérieur (20).

2. Dispositif selon la revendication 1,
**caractérisé en ce qu'**
au moins une électrode (16a, 16b, 21) fait partie d'une étiquette.

3. Dispositif selon la revendication 1,
**caractérisé en ce qu'**
au moins une électrode (16a, 16b, 21) est formée d'un film métallique collé sur la paroi.

4. Dispositif selon la revendication 1,
**caractérisé en ce qu'**
au moins l'une des électrodes extérieures (16a, 16b) est imprimée, déposée à la vapeur ou par le procédé de pulvérisation (PVD) sur la paroi (11).

5. Dispositif selon l'une des revendications 1 à 4,
**caractérisé en ce qu'**
au moins une électrode (16a, 16b, 21) est en un matériau optiquement transparent et électroconducteur, par exemple en oxyde d'indium-zinc (ITO).

6. Dispositif selon l'une des revendications 1 à 5,
**caractérisé en ce que**
l'électrode intérieure (21) est principalement en forme de haltère.

7. Dispositif selon l'une des revendications 1 à 6,
**caractérisé en ce que**
l'électrode intérieure (21) comporte deux centres (29a, 29b) reliés l'un à l'autre.

8. Dispositif selon la revendication 7,
**caractérisé en ce que**
la surface d'un centre (29a, 29b) est plus petite que la surface d'une électrode extérieure (16a, 16b), correspondante, située en regard.

9. Dispositif selon l'une des revendications 1 à 8,
**caractérisé en ce que**
l'électrode intérieure (21) a un contour formé de nombreux points de changement de direction (33a, 33b).
